# EUROPEAN PATENT APPLICATION

(11) **EP 4 741 823 A1**
(43) Date of publication of application: **13.05.2026**
(21) Application number: 25214386.2
(22) Date of filing: 07.11.2025
(51) Int. Cl.: G01N 33/38

(54) **HUMIDITY MEASURING DEVICE FOR CONCRETE CONSTRUCTIONS AND RELATED METHOD**

(30) Priority: 11.11.2024 SE 2451131
(71) Applicant: ElectroTech Kalix AB, 952 31 Kalix (SE)
(72) Inventor: HAHTO, Rickard, 952 31 KALIX (SE)
(74) Representative: Noréns Patentbyrå AB

(57) **Abstract**

A humidity measuring device (100) for measuring humidity of a construction (1) comprising a cementitious material (2) and a related method are disclosed. A casing (110) of the device (100) has a first set (111) of through-holes and a second set (112) of through-holes. A cavity (130) of the casing (110) is in fluid communication with the first and second sets (111, 112) of through-holes. A first vapor-permeable membrane (151) is arranged at the first set (111) of through-holes. A second vapor-permeable membrane (152) is arranged at the second set (112) of through-holes. At least one of the first and second vapor-permeable membranes (151, 152) is oleophobic and hydrophobic.

## Description

### TECHNICAL FIELD

The embodiments herein relate to monitoring humidity in concrete curing environments, such as newly cast concrete constructions. In particular, a humidity measurement device and a related method are disclosed.

### BACKGROUND

Measuring the humidity in concrete is essential in various construction and maintenance applications to ensure structural stability, durability, and optimal curing. Accurate humidity measurements help monitor concrete hydration during curing, and determine the appropriate timing for applying surface treatments and/or applying further constructional or surface layers. This data is also valuable in long-term monitoring of existing structures, helping to identify potential moisture-related issues that may compromise integrity over time.

However, ensuring accurate humidity measurements in concrete presents several challenges. The high moisture content of freshly mixed concrete, coupled with environmental fluctuations, makes it difficult to obtain reliable readings. Condensation, in particular, poses a significant problem, as it interferes with sensor readings, potentially leading to false data and sensor degradation. Inaccurate measurements risk premature application of surface layers or further construction, which can inadvertently trap moisture within the structure. Over time, this trapped moisture may lead to mold growth, release of volatile compounds, material degradation, and compromised structural health. In addition, release of volatile compounds can cause health problems for humans.

To prevent these issues, it is crucial to monitor humidity levels carefully to confirm that the concrete is sufficiently dry before proceeding with subsequent construction phases, such as applying surface treatments. By ensuring that excess moisture is not present, builders can mitigate the risks of mold formation and extend the lifespan of the structure, promoting a safer and more durable built environment.

EP2699901B1 discloses a measurement device for determining parameters of screeds in a building construction. The measurement device has a housing in which a moisture sensor is arranged. The moisture sensor can determine the residual moisture of the screed in a non-destructive manner. The housing has at least one interior chamber which is in communication with surroundings of the housing via at least two openings, e.g. designed as grids to prevent solids of liquid screeds from entering the chamber. The opening(s) can be closed by water vapor permeable membrane(s) arranged in the interior of the measuring device. The moisture sensor is arranged in the interior chamber. The housing of the measurement device has fastening means, by means of which the measurement device can be fixed to a carrier layer.

In view of the above, a problem may thus be how to provide reliable and/or accurate measurement device for measuring humidity in the context of concrete curing.

### SUMMARY

An object is to alleviate, or eliminate, one or more of the problems and/or disadvantages mentioned herein.

This object, or possibly other objects, can be achieved by the independent claims appended herewith.

According to an aspect, there is provided a humidity measuring device for measuring humidity of a construction comprising a cementitious material. The humidity measuring device comprises a casing having a first set of through-holes and a second set of through-holes, wherein the casing comprises a cavity that is in fluid communication with the first set of through-holes and the second set of through-holes, and a humidity sensor configured to measure water vapor content in humid air in the cavity, wherein the humidity sensor is arranged in the cavity. The first set of through-holes and the second set of through-holes are arranged at spaced positions of the casing to allow the humid air to be received, e.g., from beneath, at the humidity sensor when the humidity measuring device is installed in the construction.

Furthermore, the measuring device comprises a first vapor-permeable membrane arranged at, e.g. covering, or the like, the first set of through-holes, and a second vapor-permeable membrane arranged at, e.g. covering, or the like, the second set of through-holes. At least one of the first and second vapor-permeable membranes is oleophobic and hydrophobic.

Thanks to that at least one of the first and second vapor-permeable membranes is oleophobic gathering of the cementitious material on and/or clogging of the membrane(s) by the cementitious material can be prevented, or at least reduced. This is unexpected, since the cementitious material, such as a concrete mixture, or the like, is typically a water-based mixture, which normally can be repelled, or turned away, by a hydrophobic membrane. Since gathering at, or clogging of, the membrane(s) is reduced, the humid air is not obstructed, or at least less obstructed, when passing in and/or out of the humidity measuring device, e.g. in and/or out of the casing, and e.g. in more detail in and/or out of the cavity. Diffusion of moist, e.g. travelling by diffusion, into and/or with the humid air, e.g. due to evaporation of water within the cementitious material, can then more easily reach the humidity sensor. As a result, improved accuracy of the humidity measurements performed by the humidity measuring device is achieved.

As an example, an oleophobic side of the membrane is arranged to face away from the casing.

Again, at least one of the first and second vapor-permeable membranes is hydrophobic. In this manner, further reduction of accumulations cementitious material at the membrane(s) can be achieved. Thus, further reduction and/or further prevention of clogging of the membranes can be achieved.

In some embodiments, the cavity has a volume of less than 15 milliliters, preferably less than 10 milliliters, more preferably less than 5 milliliters. Sometimes, the volume can be about 3 milliliters, or the like. An advantage can thus be that duration of initial equilibration can be reduced as compared to when the volume of the cavity is greater than specified according to these embodiments.

In some embodiments, said at least one of the first and second vapor-permeable membranes is provided with an oleophobic coating, e.g. applied on at least one side of the membrane(s). The membrane is preferably arranged at the casing with the side with the oleophobic coating facing away from an interior of the casing, e.g. in a normal direction away from the casing.

In some embodiments, the humidity sensor is a surface mounted humidity sensor with a single flat surface that is sensible to humidity. While prior art appears to use a two-sided humidity sensor for the purpose of measuring humidity, the embodiments herein have achieved accurate and/or reliable measurements of humidity using a one-sided humidity sensor.

In some embodiments, the humidity sensor is arranged to be oriented such that the flat surface faces downwards and/or sideways, when the humidity measuring device is installed in the mold or is embedded in the construction.

Additionally, the measuring device can comprise a protective arrangement arranged at the first and second vapor-permeable membranes, thereby protecting the first and second vapor-permeable membranes from mechanical damage, e.g. due to grains in the cementitious material and/or wear before and/or during installation in the construction.

In some embodiments, the protective arrangement comprises one or more of a grating, a lattice, a grid, or the like.

In some examples, the protective arrangement is configured with a holding portion that is configured to abut the casing when the protective arrangement is mounted at the casing. Furthermore, the protective arrangement can include a first protecting portion and a second protecting portion. The protective arrangement can be configured such that the first protecting portion and the second protecting portion are spaced away from the casing when the protective arrangement is mounted at the casing. In this manner, it is facilitated that the cementitious material is allowed to flow over and cover the membrane(s), while at the same time solids and/or grains in the cementitious material do not gather and obstruct the humid air passing the humidity sensor.

In some embodiments, the first and second sets of through-holes and the cavity are arranged and configured to cause the humid air to travel vertically, or substantially vertically, at least in the vicinity of the humidity sensor.

In some embodiments, the first set of through-holes is arranged at a first side of the casing, and the second set of through-holes is arranged at a second side of the casing. The first and second sides are located oppositely to each relatively the humidity sensor. As an example, the first and second sides are opposite sides of the casing.

In some embodiments, the first set of through-holes emerges at a first outer surface of the casing. The first outer surface, or first exterior surface, can face away from the cavity.

In some embodiments, the second set of through-holes emerges at the second outer surface. The second outer surface, or second exterior surface, can face away from the cavity.

In some examples, the first outer surface faces in a first direction that is at an obtuse or right angle with respect to a second direction in which the second outer surface faces.

Sometimes, the first direction can be parallel with the second direction.

As an example, the first vapor-permeable membrane can be arranged at, e.g., covering, or the like, the first outer surface of the casing.

As an example, the second vapor-permeable membrane can be arranged at, e.g., covering, or the like, the second outer surface of the casing.

Thanks to that the first and/or second vapor permeable membrane can be arranged at the exterior of the casing, i.e., at the first and second outer surfaces of the casing, respectively, it can be avoided that blind-holes formed by e.g. through-holes in a grating and the membrane(s) are filled with cementitious materials, which in turn can clog the membrane(s).

According to a further aspect, there is provided a method for installing a humidity measuring device at a location in a mold of a concrete construction to be formed. The humidity measuring device can be according to, e.g., embodied by, or the like, the embodiments herein. The humidity measuring device is mounted, e.g. by a construction worker, a robot, or the like, in an orientation, in which a single flat surface of the humidity measuring device, the single flat surface being configured for sensing humidity, faces downwards and/or sideways, e.g., in a sensor direction that is at an angle in a range of zero to 89 degrees with respect to a vertical direction. The humidity measuring device is installed in the mold before cementitious material is deposited into the mold to form the construction.

In some embodiments, the mold is provided, e.g., constructed, build, created, or the like, e.g., by a construction worker, a robot, or the like. providing the mold.

In some embodiments, the humidity measuring device is provided, e.g., brought forward, carried, or the like, e.g., by a construction worker, a robot, or the like, e.g., to the mold, or to a location close to the mold.

In some embodiments, the range is from 0 to 80 degrees, from 0 to 65 degrees, from 0 to 55 degrees, from 0 to 45 degrees, from 0 to 35 degrees, from 0 to 25 degrees, from 0 to 15 degrees, or the like.

It can be noted that it is the moisture content of the construction, or moisture residual, that is of interest. The measurement device herein measures humidity of the air in the cavity, when enclosed by the cementitious material of the construction. Hence, when enclosed by the cementitious material any changes to humidity in the cavity can be derived from an exchange of moisture and/or vapor between the cementitious material and the cavity. Therefore, the humidity of the air in the cavity is a reliable indication of moisture in the cementitious material of the construction.

### BRIEF DESCRIPTION OF THE DRAWINGS

The various aspects of embodiments disclosed herein, including particular features and advantages thereof, will be readily understood from the following detailed description and the accompanying drawings, which are briefly described in the following.
Figure 1 is a perspective view, illustrating an overview in which an example of the measuring device is shown.
Figure 2 is a perspective view, illustrating examples of the humidity measuring device having the optional protective arrangement.
Figure 3 is an exploded perspective view, illustrating an example of the humidity measuring device.
Figure 4 is a view, illustrating a first casing portion of an example of the humidity measuring device.
Figure 5 is a perspective view, illustrating the optional protective arrangement.
Figure 6 is a perspective view, illustrating the optional protective arrangement.
Figure 7 is a view, illustrating a second side of the casing.
Figure 8 is a view, illustrating the interior of the casing.
Figure 9 is a view, illustrating a side of the casing.
Figure 10 is a view, illustrating the interior of the casing.
Figure 11 is a further view, illustrating the interior of the casing.
Figure 12 is a perspective cross-sectional view, illustrating the cavity and the through-holes.
Figure 13 is a perspective cross-sectional view, illustrating the cavity and the through-holes.
Figure 14 is a cross-sectional view, illustrating the through-holes and the channel.
Figure 15 is a plan view, illustrating where the cross-sectional view of Figure 16 is taken.
Figure 16 is a cross-sectional view, illustrating the cavity and an exemplifying through-hole.
Figure 17 is a perspective cross-sectional view, illustrating the cavity.
Figure 18 is another perspective cross-sectional view, illustrating the cavity.
Figure 19 is a flow chart, illustrating examples of the method herein.

### DETAILED DESCRIPTION

As used herein, the term "cementitious material" can refer to various building materials that can be mixed with water, or other liquids, to form a mixture or paste. Aggregates may then be added, such as cement, mortar, and limes. In other words, cementitious materials are one of the ingredients in a concrete mixture, a cement mixture, or the like. Furthermore, larger pieces within the cementitious material can be referred to as coarse aggregates, such as stones, crushed stones, pieces of stones, gravel, or slag. The coarse aggregates typically can have a diameter of about 5 mm or more, yet the coarse aggregates can be shaped in many other ways than spherical, i.e. irregular as crushed stones typically may be.

As used herein, the term "mold", "mold for cementitious material" can refer to a formwork, a shuttering, a casting mold, a concrete mold, a slipform, a form liner, a falsework, a precast form, a tunnel form, a mold board, or the like, that can receive a mixture of cementitious material, e.g., in liquid phase.

As used herein, the term "membrane(s)" can refer to at least one of the first and second vapour-permeable membranes.

As used herein, the term "oleophobic", "super-oleophobic", or the like, can refer to a property of material, e.g. the membranes herein, whose surface repels oils or other non-polar fluids or liquids. E.g. the oleophobic and/or super-oleophobic membranes herein can have a low surface energy, or low binding energy, e.g. towards non-polar fluids, whereby the surface of the membrane becomes difficult to wet. Thanks to the surface is difficult to wet, or even non-wettable, it is prevented that the membrane is clogged by the cementitious material. Thus, the membrane's ability to allow vapour to pass through the membrane is maintained, or at least less degenerated by clogging.

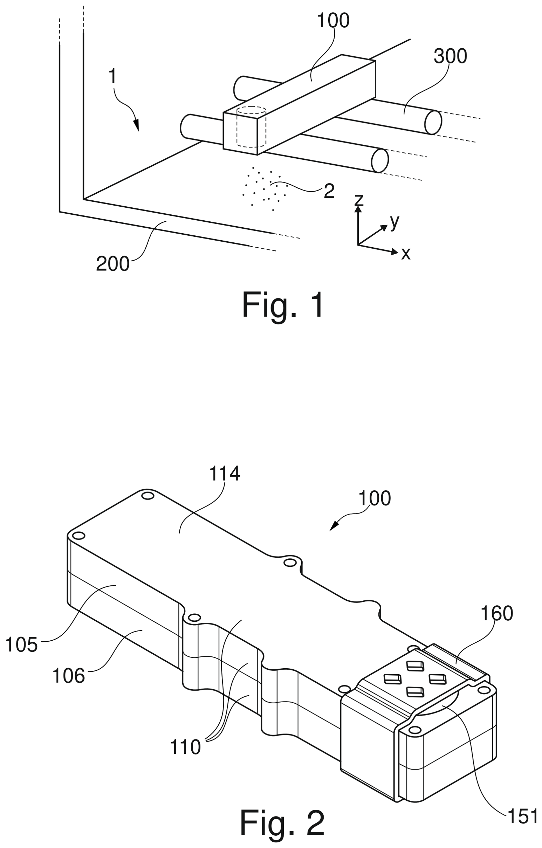

Figure 1 illustrates an overview illustrating an example of the humidity measuring device 100 for measuring humidity of a construction 1 comprising a cementitious material 2, for simplicity illustrates as a group of dots in only a portion of a mold 200. As an example, the measuring device 100 can be configured for embedding in the cementitious material 2 of the construction 1 and for obtaining a measure relating to moisture of the cementitious material 2, i.e., measuring humidity of air originating. The construction 1 can be a wall, a floor, a bridge, or a part of a bridge, a ceiling, a ramp, a building element, an element of a building, or the like.

The humidity measuring device 100 can be arranged in the mold 200, as shown in Figure 1. In the overview, two reinforcement bars 300 are shown. The reinforcement bar can be a rebar, a metal rod, a steel rebar, a glass-fiber bar, a carbon fiber rod, a composite bar, a stainless steel rod, a coated rebar, or the like.

As used herein, the terms "upwards", "downwards", "sidewards", "vertical", "vertically", and the like, are to be understood in relation to when the humidity measuring device 100 is installed in the mold 200 and/or embedded in the construction 1.

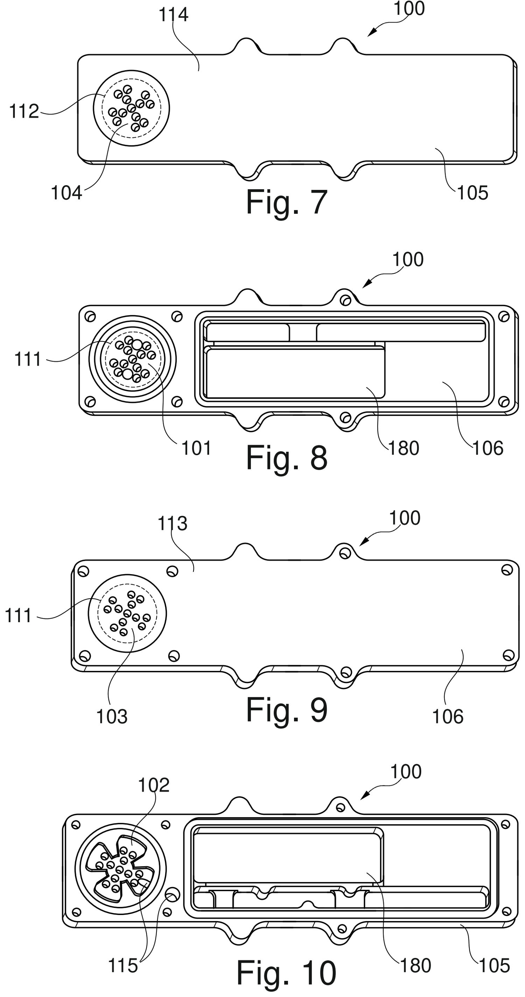

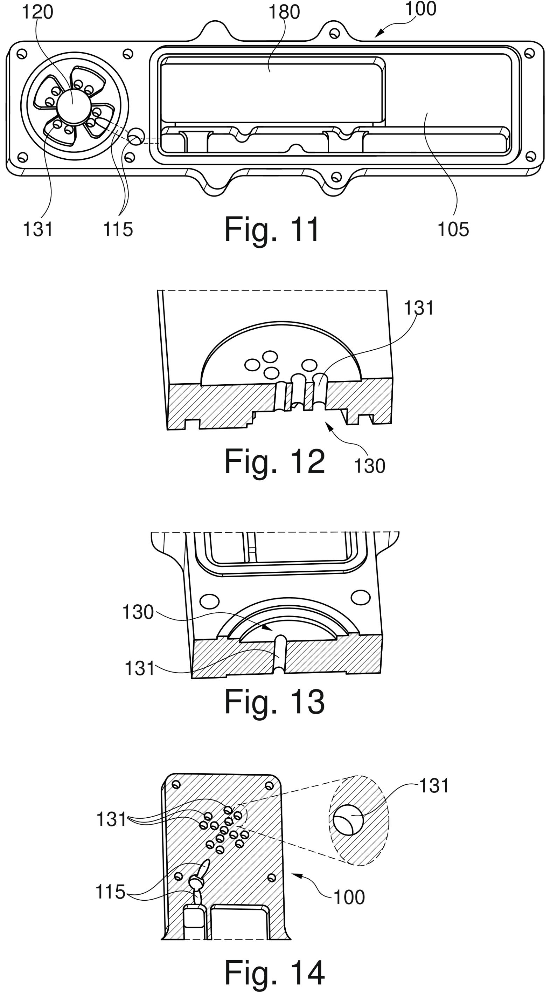

The following description is generally provided in relation to Figure 1 through Figure 14. When appropriate, a particular Figure is referenced as an example of where the described features can be seen.

The humidity measuring device 100 comprises a casing 110 having a first set of through-holes and a second set 112 of through-holes. The casing 110 can include a first casing portion 105, e.g., a first half, or the like, and a second casing portion 106, e.g., a second half, or the like. The casing 110 can comprise, such as be made of, partially be made of, include, or the like, a non-water or low water absorbable material, such as acetate plastic, polypropylene, polyethylene, polystyrene, polyvinyl chloride (PVC), polytetrafluoroethylene (PTFE), or the like.

The first set 111 of through-holes and/or the second set 112 of through-holes can include one or more through-holes 131 that penetrates the casing 110. As an example, a through-hole 131 can emerge at a first and a second inner surface 101, 102 of the casing 110 and run through the casing 110 to emerge at a first and a second outer surface 103, 104 of the casing 110. As an example, a through-hole can thus run from the first inner surface 101 to the first outer surface 103 or from the second inner surface 102 to the second outer surface 104. The first and second inner surfaces 101, 102 can preferably at least partially form a cavity 130 of the casing 110. The first inner surface 101 of the second casing portion 106 can be associated with the first set 111 of through-holes 131. The second inner surface 102 can be associated with the second set 112 of through-holes 131. As mentioned, the cavity 130 can be formed by, e.g., at least, the first and second inner surfaces 101, 102. Thus, the cavity 130 can be defined by, e.g., partially enclosed by, said first and second inner surfaces 101. 102.

Moreover, the first outer surface 103 can be associated with the second casing portion 106 and the second outer surface 104 can be associated with the first casing portion 105.

Accordingly, the casing 110 comprises the cavity 130 that is in fluid communication with the first set 111 of through-holes and the second set 112 of through-holes. Expressed differently, the cavity 130 can be in fluid communication with any existing environment, such as the cementitious material, or the like, in which the humidity measuring device 100 can be located, via the first and second sets 111, 112 of through-holes 131. This means that moist can travel, such as form a diffusive flow, in and/or out of the first and second sets 111, 112 of through-holes. Sometimes, the moist can travel into the cavity through both the first and second sets 111, 112 of through-holes, or in through one of the sets of through-holes and out through the other set of through-holes. Furthermore, it can happen that the moist travels from beneath the casing and into the cavity, and possibly out through the through-holes at the upper portion of the casing, e.g. as seen when placed in the mold and/or the construction.

Furthermore, the casing 110 comprises a humidity sensor 120 configured to measure water vapor content in humid air in the cavity 130. The humidity sensor 120 is arranged in the cavity 130 to be exposed to the humid air.

The first set 111 of through-holes and the second set 112 of through-holes can be arranged at spaced positions of the casing 110 to allow the humid air to be received at the humidity sensor 120, e.g., from beneath, when the humidity measuring device 100 is installed in the construction 1. As an example, the first set 111 of through-holes can be arranged in the second casing portion 106, and/or the second set 112 of through-holes can be arranged in the first casing portion 105. In this manner, the first set 111 of through-holes and the second set 112 of through-holes can be arranged to emerge at opposing sides of the casing 110, e.g., an upper side of the humidity sensor 100 and a lower side of the humidity sensor 100, i.e., as seen when arranged in the mold 200.

Moreover, the humidity measuring device 100 further comprises a first vapor-permeable membrane 151, arranged at, e.g., covering, the first set 111 of through-holes, as well as a second vapor-permeable membrane 152, arranged at, e.g., covering, the second set 112 of through-holes. The first vapor-permeable membrane 151 can be hydrophobic. The second vapor-permeable membrane 152 can be hydrophobic.

Additionally, the humidity measuring device 100 can comprise, e.g., is provided with, equipped with, or the like, a protective arrangement 160 arranged at the first and second vapor-permeable membranes 151, 152. In this manner, the protective arrangement 160 protects the first and second vapor-permeable membranes 151, 152 from mechanical damage, e.g. due to grains in the cementitious material 2 and/or wear before and/or during installation in the construction 1.

At least one of the first and second vapor-permeable membranes 151, 152 of the humidity measuring device 100 is oleophobic. Thanks to that said at least one of the first and second vapor-permeable membranes 151, 152 is oleophobic, accumulation of cementitious material at said at least one of the first and second vapor-permeable membranes 151, 152 and/or clogging of said at least one of the first and second vapor-permeable membranes 151, 152 is reduced.

In more detail, the fact that the membrane(s) is/are oleophobic can mean that the membrane can be configured to repel the cementitious material, in particular non-polar liquids in the cementitious material. Furthermore, as an example, the membrane can have a surface energy that prevents spreading and adhesion of the cementitious material and/or the non-polar liquids on the membrane. Explicitly, the surface energy can correspond to a contact angle of 90° or more with respect to the cementitious material and/or the non-polar liquids, thereby - as mentioned - preventing spreading and adhesion of the non-polar liquids on the membrane. See below concerning measurement of contact angles.

In some embodiments, said at least one of the first and second vapor-permeable membranes 151, 152 is provided with an oleophobic coating, whereby said at least one of the first and second vapor-permeable membranes 151, 152 becomes oleophobic. As an example, said at least one of the first and second vapor-permeable membranes 151, 152 is oleophobic by being provided with the oleophobic coating.

E.g., the coating can be applied at at least one of the membrane's 151, 152 sides, i.e. at least on a side of the membrane that faces, e.g. away from the sensor, e.g. towards the protective arrangement 160, i.e. a portion thereof that is protecting the membrane 151, 152. In more detail, said one side refers to the side of the membrane 151, 152 which can be reached, e.g., by flowing thereto, by the cementitious material. In this manner, the membrane(s) can expose all, or a large portion of its/their areas towards the cementitious material. In this manner, an exchange of moist between the cementitious material and the cavity is enhanced, e.g. as compared to when a smaller surface area of the membrane is exposed to the surrounding cementitious material.

As mentioned before, one or more of the first and second vapor-permeable membranes 151, 152 is/are hydrophobic.

In some embodiments, the cavity 130 has a volume of less than 15 milliliters, preferably less than 10 milliliters, such as three milliliters, about three milliliters, or the like.

The volume of the cavity 130 is to at least some extent dependent on that the humidity sensor 120 is sufficiently small. With examples where the volume of the cavity is small, e.g., as exemplified above, measurement of relative humidity can be performed at an exact location or depth, e.g., within the mold 200 and/or the construction 1. Furthermore, when the cavity 130 is configured to enclose such small volumes, duration of initial equilibration can be reduced. Initial equilibration can refer to when the cementitious material 2 is applied, such as filled into, poured into, or the like, in the mold 200. The initial equilibration is thus a relatively short time period as compared to drying and/or curing time before any surface layers can be safely applied, e.g., a minute compared to several hours, days or months.

In some embodiments, the humidity sensor 120 is a surface mounted humidity sensor with a single flat surface 121 that is sensible to humidity. As an example, the humidity sensor 120 is a one-sided humidity sensor, e.g., as opposed to a two-sided humidity sensor.

In some embodiments, the humidity sensor 120 is arranged to be oriented such that the flat surface faces downwards and/or sideways, when the humidity measuring device 100 is installed in the mold 200 or is embedded in the construction 1. This is elaborated on in the description of the method below.

In some embodiments, the first and second sets 111, 112 of through-holes and the cavity 130 are arranged and configured to cause the humid air to flow vertically, or substantially vertically, e.g., at least in the vicinity of the humidity sensor 120. The humidity sensor 120 can be arranged within the cavity 130 such that when the humidity measuring device 100 is located in the construction 1, the flat surface 121 of the humidity sensor 120 faces in a downward vertical direction. This means that when the humid air reaches the humidity sensor 120, the humid air can travel in a vertical direction. Then, the flat surface 121 deflects the humid air, which then flows along the flat surface 121, e.g., horizontally or substantially horizontally. Once the humid air continues past the humidity sensor 120, the humid air can again travel in the vertical direction, or a substantial vertical direction.

In some embodiments, the protective arrangement 160 comprises one or more of a grating, a lattice, a grid, or the like. In some embodiments, the protective arrangement 160 has pass-through holes 161 for receiving the cementitious material. The pass-through holes 161 are positioned in the protective arrangement 160 in the vicinity of the first and second sets 111, 112 of through-holes in the casing 110. Preferably the pass-through holes 161 form a grid, a lattice, an irregular pattern, a regular pattern, or the like, e.g., in the protective arrangement 160.

In some embodiments, the casing 110 has a first section 116, in which the cavity 130 is arranged and a second section 117, in which a processing circuit 160 is arranged. The first and second sections 116, 117 are sealed from each other to prevent water from entering the second section 117. Grooves for sealing rings are shown in the Figures. The second section 117 has a battery holding portion 180, such as a battery compartment, or the like, arranged to receive a battery 170 for powering the processing circuit 125. The humidity sensor 120 is electrically connected to the processing circuit 160. The processing circuit 125 can include control and/or communications circuitry

In some embodiments, the casing 110 comprises a channel 115 in which electrical wires are arranged in a manner to maintain sealing between the first and second sections. The electrical wires are arranged to provide electrical connection between the processing circuit 160 and the humidity sensor 120.

In some examples, the channel 115 can be omitted. Instead, a flexible circuit board can be used to electrically connect the processing circuit 125 to the humidity sensor 120.The flexible circuit board can be sealed against sealing rings or the like, when connecting the first and second sections.

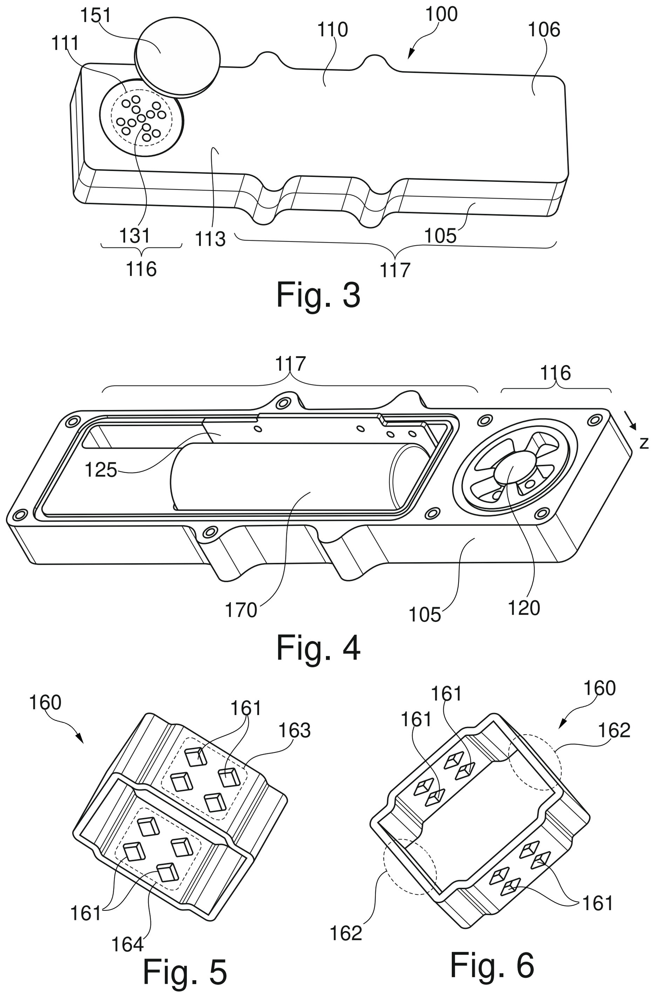

In some embodiments, a combination of the oleophobic membrane(s) 151, 152, the protective arrangement 160 and membrane(s) provided at an exterior surface of the casing 110 jointly achieves improved measurement accuracy. The configuration of the protective arrangement 160, e.g. as shown in Figure 5 and Figure 6, allows the cementitious material received through the protective arrangement 160 flow over the membrane(s) 151, 152 surface, preferably, the membrane's entire surface, where the oleophobic properties of the membrane efficiently prevents obstruction the humid air and/or clogging of the membrane(s).

In some examples, the protective arrangement 160 is configured with a holding portion 162, e.g., enclosing the humidity measuring device 100. The holding portion 162 can be configured to abut the casing 110 when the protective arrangement 160 is mounted at the casing 110. Furthermore, the protective arrangement 160 can include a first protecting portion 163 and a second protecting portion 164. The protective arrangement 160 can be configured such that that the first protecting portion 163 and the second protecting portion 164 are spaced away from the casing 110, i.e., from an exterior surface of the casing, when the protective arrangement 160 is mounted at the casing 110. In this manner, it is facilitated that the cementitious material 2 is allowed to flow over and cover the membrane(s) 151, 152, while at the same time solids and/or grains in the cementitious material 2 is prevented from, at least to some extent, to harm the membrane(s).

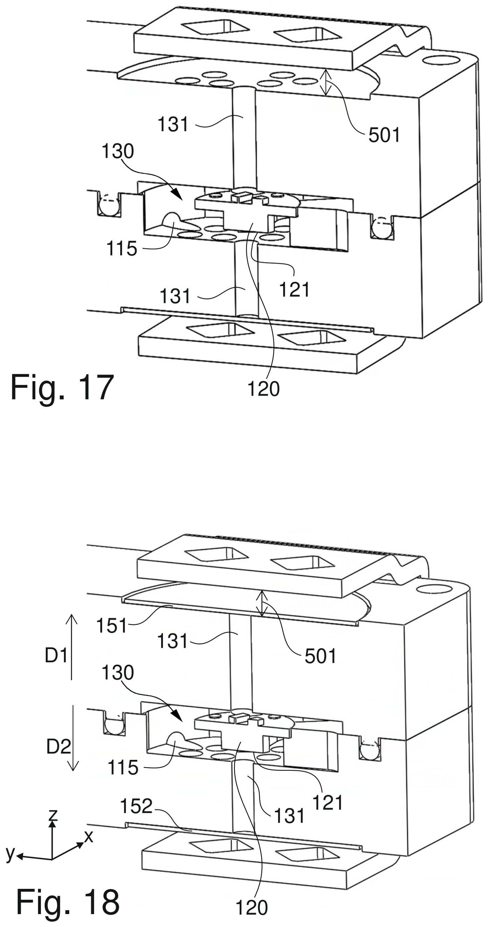

In some embodiments, the first set 111 of through-holes 131 emerges at a first outer surface 103 of the casing 110. The first outer surface 103 faces in a first direction D1 that can be at an obtuse or right angle with respect to a second direction D2 in which a second outer surface 104 faces. The second set 112 of through-holes 131 emerges at the second outer surface 104. With reference to, e.g., Figure 18, the first direction D1 can be parallel with the second direction D2. In some examples, the first and second directions align with the vertical direction Z.

The first and/or second protective portions 163, 164 can be arranged at, e.g., located in the vicinity of, or the like, the first and/or second outer surface 103, 104, respectively. Accordingly, the first and/or second protective portions 163, 163 can match the first and/or second outer surfaces 103, 104, whereby the membrane(s) are protected, at least to some extent.

It shall be understood that moist can of course enter the cavity 130 when the through-holes 131 are tilted relatively the vertical direction Z. As an example, there can be such tilt when the first and second directions D1, D2 form the obtuse or right angle as explained above. Furthermore, the through-holes 131 do not necessarily have to be straight, e.g., cylindrical. The through-holes 131 can follow a curved path, or the like. A cross-section of the through-holes 131 can further have any desired shape and/or form, such as triangular, circular, polygonal, or the like.

As mentioned above, the first protecting portion 163 can be spaced away from the casing 110, i.e., spaced away from the first outer surface 103, e.g., by 1-5 mm, by 2-4 mm, or the like. Furthermore, the second protecting portion 164 can be spaced away from the casing 110, i.e., spaced away from the second outer surface 104, e.g., by 1-5 mm, by 2-4 mm, or the like. See distance 501 in Figure 17 and Figure 18 (shown for only one of the protecting portions).

An advantage with that the first and/or second protecting portions 163, 164 are spaced away from the membrane(s) can be that a free, unobstructed side of the membrane(s) faces the cementitious material. E.g. as compared to prior art, in which the grid and the membrane form blind-holes, which can become obstructive to passing humid air and/or possibly cause clogging of the membrane(s).

In some embodiments, the first set 111 of through-holes is arranged at a first side 113 of the casing 110, and the second set 112 of through-holes is arranged at a second side 114 of the casing 110. The first and second sides 113, 114 are located oppositely to each relatively the humidity sensor 120. The first and second sides can be opposite sides of the casing 110. As an example, the first outer surface can be a portion of the first side and/or the second outer surface can be a portion of the second side, or vice versa.

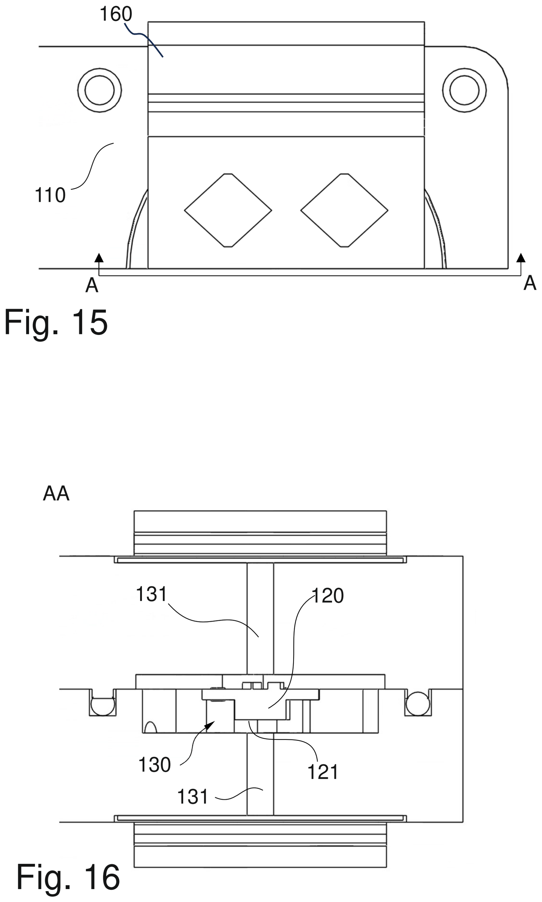

Figure 15 shows section A-A of which a cross-sectional view is illustrated in Figure 16.

Figure 16 illustrates a cross-section of the cavity 130 seen in the normal direction of the plane AA illustrated in Figure 15. As an example, the flat surface 121 of the humidity sensor 120 faces downwards, again, when mounted to be cast into the construction 1.

Figure 17 is a perspective view of the cross-section shown in Figure 16. In Figure 17, the membranes are not shown, such at to reveal the through-holes 131 in the casing, i.e. in the upper portion of the casing 110. Moreover, it is clear that the cavity 130 houses the humidity sensor 120, which can be mounted on a printed circuit board.

Figure 17 and Figure 18 also shows that the channel 115 emerges into the cavity 130.

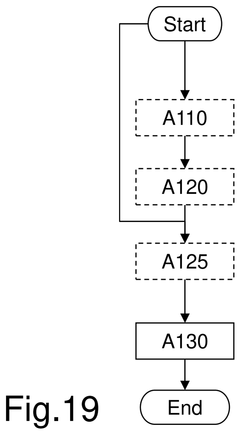

Figure 19 shows an exemplifying method for installing a humidity measuring device 100 at a location in the mold 200 of the concrete construction 1 to be formed.

One or more of the following actions can be performed, e.g. in the order as below or in any other suitable order when applicable.

### Action A110

The mold 200 can be provided, such as built, created, generated, formed, or the like. As an example, one or more construction workers and/or a machine, such as a robot, or the like, can build the mold 200 which is to be used when creating the construction 1.

### Action A120

The humidity measuring device 100 can be provided, such as accessed, brought, or the like, at, e.g., to the mold 200.

### Action A125

Preferably before action A130, at least one side of the first and/or second membrane(s) can be made oleophobic, e.g., unless the membrane(s) not already is/are oleophobic. As an example, the membrane(s) can be made oleophobic by that an oleophobic coating is provided, such as applied, sprayed, poured, or the like, at said at least one side. Sometimes, the membrane(s) can be dipped into a liquid that can form the coating on the membrane(s). When the membrane is mounted at the humidity measuring device 100, said at least one side can face away from the cavity.

On the market, there exist various sprays, films, liquids, or the like, that can be applied to a surface in order to make the surface oleophobic.

### Action A130

In order to measure the moisture content of the construction during hardening process, the humidity measuring device 100 is mounted in an orientation, in which the single flat surface 121 of the humidity measuring device 100 faces downwards and/or sideways. As explained above, the single flat surface 121 is the only surface of the humidity sensor that can, i.e. is configured for, sensing humidity.

### Measurement of contact angle

In the following some examples of methods of how to measure the contact angle in order to obtain a measure of surface energy are briefly identified.

Sessile Drop Method is a method, where a droplet of liquid is placed on a flat surface, and the contact angle is measured using a goniometer or an image analysis system. This method provides a static contact angle measurement.

Dynamic Contact Angle Measurement (Advancing and Receding Angles) involves gradually increasing (advancing) or decreasing (receding) the volume of a droplet on a surface to measure the dynamic behavior of the contact angle. This method captures both advancing and receding angles, which can indicate surface heterogeneity or roughness.

With Tilted Plate Method, a droplet is placed on a surface, and the surface is gradually tilted until the droplet begins to slide. The contact angle is measured just before movement, and this provides information about both the contact angle and the droplet's sliding behavior.

Captive Bubble Method is used for surfaces submerged in a liquid, this method involves introducing an air bubble against the underside of the surface, then measuring the angle of contact between the bubble and the solid. This is particularly useful for hydrophobic surfaces in aqueous environments.

Wilhelmy Plate Method is an indirect method, where a thin plate of the material is dipped into and withdrawn from a liquid. By measuring the force on the plate due to the liquid's surface tension, the contact angle can be calculated.

Drop Shape Analysis (DSA) uses a high-resolution camera to capture the shape of a droplet in real-time, allowing the measurement of contact angles with advanced image analysis. This method is particularly useful for time-dependent changes in contact angle on complex or non-uniform surfaces.

As an example, the humidity sensor can face in a sensor direction, that e.g., can be at an angle in a range of zero to 89 degrees with respect to a vertical direction. In this manner, the humidity measuring device 100 is installed, such as mounted, put, placed, located, or the like, in the mold 200 before the cementitious material 2 is deposited into the mold 200 in order to form the construction 1.

In some embodiments, the range is from 0 to 45 degrees, from 0 to 35 degrees, from 0 to 25 degrees, from 0 to 15 degrees, or the like.

According to various examples, the orientation of the flat surface 121 of the humidity sensor 120 can be such that a normal direction of the flat surface 121 corresponds to a sensor direction.

The sensor direction can be downwards and at an angle in a range of zero to 89 degrees with respect to a vertical direction, i.e., when the humidity measuring device 100 is installed in the construction 1, e.g., preferably towards the first set 111 of through-holes. In this manner, it can be avoided that condensed water accumulates on the flat sensor surface 121.

Any feature disclosed for one example and/or embodiment may be combined with one or more features disclosed for one or more other examples and/or embodiments without departing from the scope herein.

As used herein, a set of items refers to one or more such items.

As used herein, the term "arrange" can, unless otherwise evident from the context, refer to how one feature is located, or positioned, relatively another feature.

As used herein, the term "configured" can, unless otherwise evident from the context, refer to a shape and/or a form and/or an appearance and/or a property of the feature being described.

## Claims

1. A humidity measuring device (100) for measuring humidity of a construction (1) comprising a cementitious material (2), wherein the humidity measuring device (100) comprises:
a casing (110) having a first set (111) of through-holes and a second set (112) of through-holes, wherein the casing (110) comprises
a cavity (130) that is in fluid communication with the first set (111) of through-holes and the second set (112) of through-holes, and
a humidity sensor (120) configured to measure water vapor content in humid air in the cavity (130), wherein the humidity sensor (120) is arranged in the cavity (130),
wherein the first set (111) of through-holes and the second set (112) of through-holes are arranged at spaced positions of the casing (110) to allow the humid air to be received at the humidity sensor (120) when the humidity measuring device (100) is installed in the construction (1),
wherein the measuring device (100) comprises
a first vapor-permeable membrane (151) arranged at the first set (111) of through-holes, and
a second vapor-permeable membrane (152) arranged at the second set (112) of through-holes, wherein the humidity measuring device (100) is **characterized by** that
at least one of the first and second vapor-permeable membranes (151, 152) is oleophobic and hydrophobic.

2. The humidity measuring device (100) according to claim 1, wherein the cavity (130) has a volume of less than 15 milliliters, preferably less than 10 milliliters.

3. The humidity measuring device (100) according to any one of the preceding claims, wherein said at least one of the first and second vapor-permeable membranes (151, 152) is provided with an oleophobic coating,

4. The humidity measuring device (100) according to any one of the preceding claims, wherein the humidity sensor (120) is a surface mounted humidity sensor with a single flat surface (121) that is sensible to humidity.

5. The humidity measuring device (100) according to claim 4, wherein the humidity sensor (120) is arranged to be oriented such that the flat surface (121) faces downwards and/or sideways, when the humidity measuring device (100) is installed in the mold (200) or is embedded in the construction (1).

6. The humidity measuring device (100) according to any one of the preceding claims, wherein the humidity measuring device (100) comprises
a protective arrangement (160) arranged at the first and second vapor-permeable membranes (151, 152), thereby protecting the first and second vapor-permeable membranes (151, 152) from mechanical damage and/or wear before and/or during installation in the construction (1).

7. The humidity measuring device (100) according to claim 6, wherein the protective arrangement (160) has pass-through holes (161) for receiving the cementitious material (2), wherein the pass-through holes (161) are located in the protective arrangement (160) in the vicinity of the first set (111) of through-holes and/or the second set (112) of through-holes, wherein preferably the pass-through holes (161) form a grid, a lattice, an irregular pattern, or a regular pattern.

8. The humidity measuring device (100) according to any one of the preceding claims, wherein the first and second sets (111, 112) of through-holes and the cavity (130) are arranged and configured to cause the humid air to flow vertically, or substantially vertically, at least in the vicinity of the humidity sensor (120).

9. The humidity measuring device (100) according to any one of the preceding claims, wherein the first set (111) of through-holes emerges at a first outer surface (103) of the casing (110), wherein the first outer surface (103) faces in a first direction (D1) that is at an obtuse or right angle with respect to a second direction (D2) in which a second outer surface (104) faces, wherein the second set (112) of through-holes emerges at the second outer surface (104), or wherein the first direction (D1) is parallel with the second direction (D2).
